# EUROPEAN PATENT SPECIFICATION

(11) **EP 3 191 991 B1**
(45) Date of publication and mention of the grant of the patent: **13.01.2021**
(21) Application number: 15778726.8
(22) Date of filing: 08.09.2015
(51) Int. Cl.: G16H 15/00, G06F 16/56

(54) **IMAGE REPORT ANNOTATION IDENTIFICATION**
IDENTIFIZIERUNG VON BILDBERICHTANMERKUNGEN
IDENTIFICATION D'ANNOTATION DE RAPPORT D'IMAGE

(30) Priority: 10.09.2014 US 201462048295 P
(43) Date of publication of application: 19.07.2017
(73) Proprietor: Koninklijke Philips N.V., 5656 AG Eindhoven (NL)
(72) Inventor: LEE, Michael Chun-chieh, NL-5656 AE Eindhoven (NL)
(74) Representative: de Haan, Poul Erik
(86) International application number: PCT/IB2015/056866
(87) International publication number: WO 2016/038535

(56) References cited:
- US-A1- 2012 041 779
- Anonymous: "BI-RADS - Wikipedia, the free encyclopedia", , 16 June 2014 (2014-06-16), XP055232103, Retrieved from the Internet: URL:https://en.wikipedia.org/w/index.php?t itle=BI-RADS&oldid=613159796 [retrieved on 2015-11-27]

## Description

### FIELD OF THE INVENTION

The following generally relates to determining an annotation for an electronically formatted image report based on previously annotated images.

### BACKGROUND OF THE INVENTION

Structured reporting is commonly used to capture descriptive information about tissue of interest (e.g., oncologic lesions) in medical imaging. With structured reporting, a radiologist labels tissue of interest in images using a standardized set of text annotations, which describe the tissue shape, orientation, location, and/or other characteristics in a manner that can be more easily interpreted by others who are familiar with the annotation nomenclature.

For example, in breast imaging, the Breast Imaging Reporting and Data System (BI-RADS) is a standard developed by the American College of Radiology. According to the standard, lesions evaluated on MRI should be described by shape (round, oval, lobular, irregular), margin (smooth, irregular, spiculated), enhancement (homogeneous, heterogeneous, rim enhancing, dark internal septation, enhancing internal septation, central enhancement), and other categories.

Similarly, in breast ultrasound, masses should be annotated as to their shape (oval, round, irregular), orientation (parallel, not parallel), margin (circumscribed, indistinct, angular, microlobulated, spiculated), and other categories. Similar systems exist or are being considered for other organs, such as lung. With such standards, a radiologist reviews the image and selects text annotations based on his or her observations and understanding of the definitions of the annotation terms.

A basic approach to structured reporting includes having a user directly select text annotations for an image or finding. This may be simply implemented as, e.g. a drop-down menu from which a user chooses a category via a mouse, touchscreen, keyboard, and/or other input device. However, such an approach is subject to the user's expertise and interpretation of the meaning of those terms. An alternative approach to structured reporting is visual reporting.

With visual reporting, the drop-down list of text is replaced with example images (canonical images) from a database, and the user selects annotations aided by example images. For example, instead of selecting just the term "spiculated", the user may select an image showing example spiculated tissue from a group of predetermined fixed images. This reduces subjectivity because the definition of the structured annotation is given by the image rather than the textual term.

This visual image annotation aids in ensuring that all users have a common understanding of the terminology. However, the example images are fixed (i.e., the same canonical "spiculated" image is always shown), and there can be a wide variability in certain tissue such as lesions. As such, the canonical examples may not be visually similar to the current image. For example, even if the current patient image is "spiculated", it may not sufficiently closely resemble the canonical "spiculated" image to be considered a match. US2012041779 discloses a clinical decision support (CDS) system that has a case grouping sub-system which includes graphical user interface (GUI) which simultaneously displays data representing patient cases, and enables the user to group selected patient cases into clinically related groups. Optionally, the case grouping sub-system further includes a case groupings report generator that generates a human-readable report of the case groupings to provide a written record of the clinically related groups, or for optional review by other physicians or other skilled human medical diagnosticians, or for other purposes.

Independent claims of the present application differ from the prior art above in the following features:
identifying, for each of a plurality of predetermined annotations, based on the determined similarities, from the set of previously annotated medical images the image with said each predetermined annotation and with the greatest similarity to the medical input image;
visually displaying, for each of the plurality of predetermined annotations, the identified image for said each predetermined annotation along with said each predetermined annotation; receiving an input signal identifying one of the displayed images and the displayed predetermined annotations; annotating the medical input image with the displayed image identified by the input signal;
including the displayed annotation identified by the input signal in a report;

### SUMMARY OF THE INVENTION

The invention is defined by the appended claims. The examples, aspects and embodiments not falling within the scope of the claims are for reference only.

### BRIEF DESCRIPTION OF THE DRAWINGS

The invention may take form in various components and arrangements of components, and in various steps and arrangements of steps. The drawings are only for purposes of illustrating the preferred embodiments and are not to be construed as limiting the invention.
FIGURE 1 schematically illustrates an example computing system with a report module.
FIGURE 2 schematically illustrates an example of report module.
FIGURE 3 illustrates an example display showing best matched images for multiple different annotation types.
FIGURE 4 illustrates an example method for generating a report with an annotation.

### DETAILED DESCRIPTION OF EMBODIMENTS

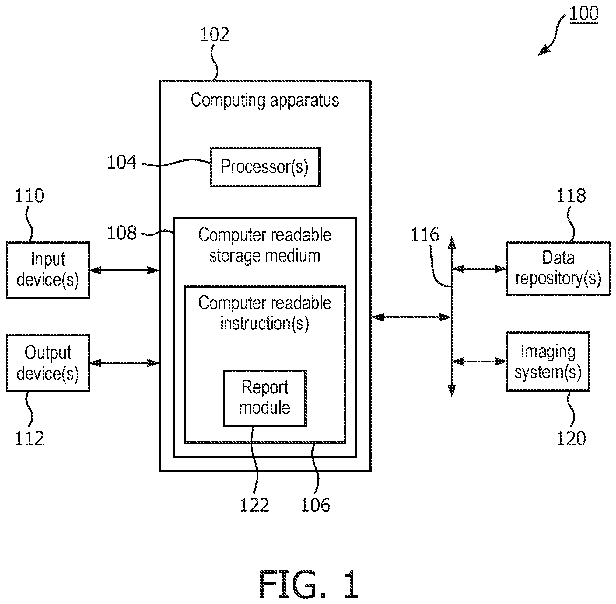

FIGURE 1 illustrates a system 100 with a computing apparatus 102 that includes at least one processor 104, which executes one or more computer readable instructions 106 stored in computer readable storage medium 108, which excludes transitory medium and include physical memory and/or other non-transitory storage medium. The processor 104 can additionally or alternatively execute one or more computer readable instructions carried by a carrier wave, a signal or other transitory medium.

The computing apparatus 102 receives information from one or more input devices 110 such as a keyboard, a mouse, a touch screen, etc. and/or conveys information to one or more output devices 112 such as one or more display monitors. The illustrated computing apparatus 102 is also in communication with a network 116 and one or more devices in communication with the network such as at least one data repository 118, at least one imaging system 120, and/or one or more other devices.

Examples of data repositories 118 include, but are not limited to, a picture archiving and communication system (PACS), a radiology information system (RIS), a hospital information system (HIS), and an electronic medical record (EMR). Examples of imaging systems 120 include, but are not limited to, a computed tomography (CT) system, a magnetic resonance (MR) system, a positron emission tomography (PET) system, a single photon emission computed tomography (SPECT) system, an ultrasound (US) system, and an X-ray imaging system.

The computing apparatus 102 can be a general purpose computer or the like located at a physician's office, a health care facility, an imaging center, etc. The computing apparatus 102 at least includes software that allows authorized personnel to generate electronic medical reports. The computing apparatus 102 can convey and/or receive information using formats such as Health Level Seven (HL7), Extensible Markup Language (XML), Digital Imaging and Communications in Medicine (DICOM), and/or one or more other format(s).

The at least one computer readable instruction 106 includes a report module 122, which, when executed by the at least one processor 104 generates, in an electronic format, a report, for an input image to be annotated, that includes an annotation. As described in greater detail below, the report module 122 determines the annotation based on the input image to be annotated and a set of previously acquired and annotated images of other patients. In one instance, the final report includes an annotation corresponding to an image that visually matches tissue of interest in the input image better than a fixed example image with a generic representation of the tissue of interest.

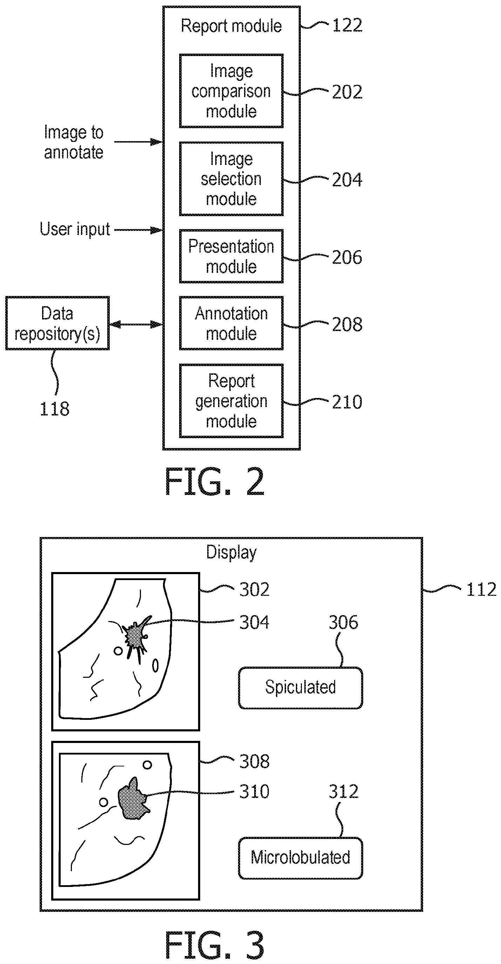

FIGURE 2 schematically illustrates an example of the report module 122.

The report module 122 receives, as input, an image (of a subject or object) to be annotated. The input image can be from the imaging system(s) 120, the data repository(s) 118, and/or other device. In this example, the input image is a medical image, for example, a MRI, CT, ultrasound, mammography, x-ray, SPECT, or PET image. However, in a variation, the input image can be a non-medical image, such as an image of an object in connection with non-destructive testing, security screening (e.g., airport), and/or other non-medical application.

In this example, the report module 122 has access to the data repository(s) 118. It is to be appreciated that the report module 122 may have access to other data storage that stores previously acquired and annotated images, including cloud based storage, distributed storage, and/or other storage. The data repository(s) 118 includes, at least, a database of images of other patients for which annotations have already been created. Example image formats for such images include DICOM, JPG, PNG and/or other electronic image format.

In one instance, the data repository(s) 118 is a separately held curated database where images have been specifically reviewed for use in the application. In another instance, the data repository(s) 118 is a database past patients at a medical institution, for example, as stored in a PACS. Other data repositories are also contemplated herein. In this example, the data repository(s) 118 includes the image and the annotation. In another example, the image and the annotation are stored on separated devices.

Generally, the data repository(s) 118 includes at least one image representing each of the available annotations. For example, in one instance a set of available annotations includes margin annotations (e.g., "spiculated" or "circumscribed"), shape annotations (e.g., "round" or "irregular"), and/or one or more other annotations. For this set, the data repository(s) 118 includes at least one spiculated example image, at least one circumscribed example image, at least one round example image, and at least one irregular example image.

The report module 122 includes an image comparison module 202. The image comparison module 202 determines a similarity metric between the input image and one or more of the previously annotated images in the data repository(s) 118.

For the comparison, in one instance, the report module 122 receives a user input identifying a point or sub-region within the input image to identify tissue of interest in the input image to annotate. In another instance, the entire input image, rather than just the point or the sub-region of the input image, is to be annotated. In the later instance, the user input is not needed.

For the comparison, in one example, the identified portion or the entire two images (i.e., the input image and the previously annotated image) are compared. For this, the portion is first segmented using known and/or other approaches. Quantitative features are then computed using known and/or other approaches, generating numerical features descriptive of the size, position, brightness, contrast, shape, texture of the object and its surroundings, yielding a "feature vector". The two feature vectors are then compared using, e.g. a Euclidean distance measure, with shorter distances representing more similar objects.

In another example, the images are compared in a pixel-wise (or voxel-wise, or sub-group of pixel or voxel-wise) approach such as sum-of-squared difference, mutual information, normalized mutual information, cross-correlation, etc. In the illustrated example, a single image comparison module (e.g., the image comparison module 202) performs all of the comparisons. In another example, there is a separate image comparison module for each annotation, at least one image comparison module for two or more comparisons and at least one other image comparison module for a different comparison, etc.

The report module 122 further includes an image selection module 204. The image selection module 204 selects a candidate image for each annotation.

In one instance, a single most similar image is selected. This can be done by identifying the image with a highest similarity measure and the requisite annotation. For example, where a lesion is described by margin ("spiculated" or "circumscribed") and shape ("round" or "irregular"), the most similar "spiculated" lesion is identified, the most similar "circumscribed" lesion is identified, the most similar "round" lesion, and the most similar "irregular" lesion. There may be overlap, e.g. the most similar circumscribed lesion may also be the most similar round lesion.

In another instance, a set of similar images is identified where each set consists of at least one image. This may be achieved by selecting a subset of images (from the data repository(s) 118) with a given annotation where a similarity is greater than a pre-set threshold. Alternatively, this may be done by selecting a percentage of cases. For example, if similarity is measured on a 0-to-1 scale, with the above example, all spiculated lesions with a similarity greater than 0.8 may be chosen, or the 5% of spiculated lesions that have the highest similarity may be chosen. This is repeated for each annotation type.

The report module 122 further includes a presentation module 206. The presentation module 206 visually presents (e.g., via a display of the output device(s) 112) each annotation and at least one most similar image for each annotation. An example is shown in FIGURE 3, which shows an image 302 with spiculated tissue 304 for the annotation spiculated 306, and an image 308 with microlobulated tissue 310 for the annotation microlobulated 312. In another instance, multiple images may be shown for an annotation. For example, instead of showing a single image representing the annotation "spiculated" 306 in FIGURE 3, multiple images are shown for the annotation "spiculated" 306.

The report module 122 further includes an annotation module 208. The annotation module 208, in response to receiving a user input identifying one of the displayed images and/or annotations, annotates the input image with the displayed image. The visually presented images (e.g., FIGURE 3) aid the user in selecting the correct annotation. The user can select an image, e.g. by clicking on a nearby button, clicking on the image, and/or similar operation.

The report module 122 further includes a report generation module 210. The report generation module 210 generates, in an electronic format, a report for the input image that includes the user selected annotation spiculated 306. In a variation, the report is a visual report, which further includes the identified annotated image 302 as a visual image annotation.

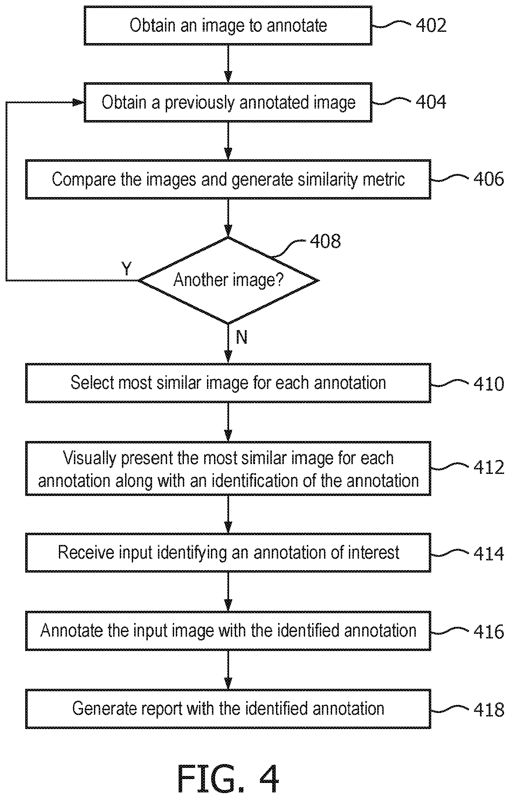

FIGURE 4 illustrates an example flow chart in accordance with the disclosure herein.

It is to be appreciated that the ordering of the acts in the methods described herein is not limiting. As such, other orderings are contemplated herein. In addition, one or more acts may be omitted and/or one or more additional acts may be included.

At 402, an image to annotate is obtained.

At 404, a previously annotated image is obtained.

At 406, a similarity metric is determined between the two images.

At 408, it is determined if another previously annotated image is to be compared.

In response to there being another previously annotated image to compare, acts 404 through 408 are repeated.

At 410, in response to there not being another previously annotated image to compare, a most similar image is identified for each annotation based on the similarity metric.

At 412, the most similar previously annotated image for each annotation, along with an identification of the corresponding annotation, is visually presented.

At 414, an input indicative of a user identified previously annotated image and/or annotation is received.

At 416, the input image is annotated with the identified annotation.

At 418, a report, in electronic format, is generated for the input image with the identified annotation, and optionally, the identified image as a visual image annotation.

The above may be implemented by way of computer readable instructions, encoded or embedded on computer readable storage medium, which, when executed by a computer processor(s), cause the processor(s) to carry out the described acts. Additionally or alternatively, at least one of the computer readable instructions is carried by a signal, carrier wave or other transitory medium.

The invention has been described with reference to the preferred embodiments. Modifications and alterations may occur to others upon reading and understanding the preceding detailed description. It is intended that the invention be constructed as including all such modifications and alterations insofar as they come within the scope of the appended claims or the equivalents thereof.

## Claims

1. A computer-implemented method for creating an electronically formatted medical image report with an image annotation, comprising:
receiving a medical input image, of a patient, to be annotated
comparing the medical input image with a set of previously annotated medical images to determine similarities between the medical input image and each image in the set of previously annotated medical images;
identifying, for each of a plurality of predetermined annotations, based on the determined similarities, from the set of previously annotated medical images the image with said each predetermined annotation and with the greatest similarity to the medical input image;
visually displaying, for each of the plurality of predetermined annotations, the identified image for said each predetermined annotation along with said each predetermined annotation;
receiving an input signal identifying one of the displayed images and the displayed predetermined annotations;
annotating the medical input image with the displayed image identified by the input signal; and
generating, in an electronic format, a report for the medical input image that includes the displayed annotation identified by the input signal.

2. The method of claim 1, wherein the identifying identifies, for each of the plurality of predetermined annotations, a set of one or more previously annotated medical images with the greatest similarity to the medical input image.

3. The method of claim 2, wherein the two or more previously annotated medical images each have a similarity greater than a predetermined threshold similarity level.

4. The method of claim 2, wherein the two or more previously annotated medical images each have a similarity in a predetermined percentage range of similarities.

5. The method of any of claims 1 to 4, wherein the set of previously annotated medical images includes previously annotated medical images for other patients.

6. The method of any of claims 1 to 5, wherein the set of previously annotated medical images includes at least one annotated medical image corresponding to each of the plurality of predetermined annotations.

7. The method of any of claims 1 to 6, wherein an entirety of the medical input image and an entirety of each image of the set of previously annotated medical images are compared.

8. The method of claim 7, wherein images are compared one of pixel-wise, voxel-wise, sub-group of pixel-wise, or sub-group of voxel-wise.

9. The method of any of claims 1 to 6, further comprising:
receiving a signal indicating a sub-region of the medical input image, wherein only the sub-region of the medical input image and a corresponding sub-region of each image of the set of previously annotated medical images is compared.

10. The method of claim 9, further comprising:
segmenting the sub-region of the medical input image and the corresponding sub-region of each image of the set of previously annotated medical images; and
comparing the segmented sub-region of the medical input image and the segmented sub-region of each image of the set of previously annotated medical images.

11. The method of any of claims 7 to 10, further comprising:
generating a quantitative feature vector for each of the medical input image and each image of the set of previously annotated medical images, wherein the comparison includes comparing the quantitative feature vectors.

12. The method of claim 11, wherein the quantitative feature vector includes numerical features descriptive of one of more of a size, a position, a brightness, a contrast, a shape, or a texture.

13. A computing apparatus (102), comprising:
a first input device (110) that receives a medical input image, of a patient, to be annotated;
a processor (104) that compares the medical input image with a set of previously annotated medical images to determine similarities between the medical input image and each image in the set of previously annotated medical images, and identifies, for each of a plurality of predetermined annotations, based on the determined similarities, from the set of previously annotated medical images the image with said each predetermined annotation and with the greatest similarity to the medical input image;
a display (112) that visually displays, for each of the plurality of predetermined annotations, the identified image for said each predetermined annotation along with said each predetermined annotation; and
a second input device that receives an input signal identifying one of the displayed images and the displayed predetermined annotations, wherein the processor annotates the medical input image with the displayed image identified by the input signal and generates, in an electronic format, a report for the medical input image that includes the displayed annotation identified by the input signal.

14. The computing apparatus of claim 13, wherein the processor identifies, for each of the plurality of predetermined annotations, a set of one or more previously annotated medical images with the greatest similarity to the medical input image.

15. A computer readable storage medium (108) encoded with computer readable instructions, which, when executed by a processor (104), causes the processor to implement the method according to claim 1.

## Patentansprüche

1. Eine auf einem Computer auszuführende Methode zum Erstellen eines elektronisch formatierten medizinischen Bildberichts mit einer Bildanmerkung, die folgende Schritte umfasst:
Abrufen des medizinischen Eingangsbilds eines Patienten, das mit Anmerkungen versehen werden soll,
Vergleichen des medizinischen Eingangsbilds mit einer Reihe von zuvor mit Anmerkungen versehenen medizinischen Bildern, um Ähnlichkeiten zwischen dem medizinischen Eingangsbild und den einzelnen Bildern der Reihe der zuvor mit Anmerkungen versehenen medizinischen Bildern zu ermitteln;
Ermitteln des Bilds mit der jeweiligen vorab festgelegten Anmerkung sowie mit der größten Ähnlichkeit mit dem medizinischen Eingangsbild für die einzelnen vorab festgelegten Anmerkungen beruhend auf den ermittelten Ähnlichkeiten aus der Reihe der zuvor mit Anmerkungen versehenen medizinischen Bilder;
visuelles Darstellen des ermittelten Bilds für die einzelnen vorab festgelegten Anmerkungen gemeinsam mit den einzelnen vorab festgelegten Anmerkungen;
Abrufen eines Eingangssignals, das eines der angezeigten Bilder und die angezeigten vorab festgelegten Anmerkungen angibt;
Anmerken des medizinischen Eingangsbilds mit dem anhand des Eingangssignals ermittelten angezeigten Bild; und
Generieren eines elektronischen Berichts für das medizinische Bild, der die angezeigte, anhand des Eingangssignals ermittelte Anmerkung enthält.

2. Die Methode gemäß Anspruch 1, wobei beim Ermitteln für jede der vorab festgelegten Anmerkungen eine Reihe mit mindestens einem zuvor mit Anmerkungen versehenen medizinischen Bild mit der größten Ähnlichkeit mit dem medizinischen Eingangsbild ermittelt wird.

3. Die Methode gemäß Anspruch 2, wobei die mindestens zwei zuvor mit Anmerkungen versehenen medizinischen Bilder jeweils eine Ähnlichkeit aufweisen, die größer ist als ein vorab festgelegter Ähnlichkeitsschwellenwert .

4. Die Methode gemäß Anspruch 2, wobei die mindestens zwei zuvor mit Anmerkungen versehenen medizinischen Bilder jeweils eine Ähnlichkeit in einem vorab festgelegten prozentualen Ähnlichkeitsbereich aufweisen.

5. Die Methode gemäß einem der Ansprüche 1 bis 4, wobei die Reihe der zuvor mit Anmerkungen versehenen medizinischen Bilder zudem zuvor mit Anmerkungen versehene medizinische Bilder für andere Patienten umfasst.

6. Die Methode gemäß einem der Ansprüche 1 bis 5, wobei die Reihe der zuvor mit Anmerkungen versehenen medizinischen Bilder mindestens ein mit Anmerkungen versehenes medizinisches Bild umfasst, das den einzelnen vorab festgelegten Anmerkungen entspricht.

7. Die Methode gemäß einem der Ansprüche 1 bis 6, wobei die Gesamtheit des medizinischen Eingangsbilds mit der Gesamtheit aller Bilder der Reihe der zuvor mit Anmerkungen versehenen medizinischen Bilder verglichen wird.

8. Die Methode gemäß Anspruch 7, wobei die Bilder entweder pixelweise, voxelweise, nach Untergruppen von Pixeln oder nach Untergruppen von Voxeln verglichen werden.

9. Die Methode gemäß einem der Ansprüche 1 bis 6, wobei diese zudem folgenden Schritt umfasst:
Abrufen eines Signals, das eine Unterregion des medizinischen Eingangsbilds angibt, wobei nur die Unterregion des medizinischen Eingangsbilds mit der entsprechenden Unterregion der einzelnen Bilder der Reihe der zuvor mit Anmerkungen versehenen medizinischen Bilder verglichen wird.

10. Die Methode gemäß Anspruch 9, wobei diese zudem folgende Schritte umfasst:
Segmentieren der Unterregion des medizinischen Eingangsbilds und der entsprechenden Unterregionen der einzelnen Bilder der Reihe der zuvor mit Anmerkungen versehenen medizinischen Bilder; und
Vergleichen der segmentierten Unterregion des medizinischen Eingangsbilds mit den segmentierten Unterregionen der einzelnen Bilder der Reihe der zuvor mit Anmerkungen versehenen medizinischen Bilder.

11. Die Methode gemäß einem der Ansprüche 7 bis 10, wobei diese zudem folgenden Schritt umfasst:
Generieren eines quantitativen Merkmalsvektors für die einzelnen medizinischen Eingangsbilder und die Bilder der Reihe der zuvor mit Anmerkungen versehenen medizinischen Bilder, wobei der Vergleich das Vergleichen der quantitativen Merkmalsvektoren umfasst.

12. Die Methode gemäß Anspruch 11, wobei der quantitative Merkmalsvektor mindestens eines der folgenden numerischen Merkmale enthält: Größe, Position, Helligkeit, Kontrast, Form oder Textur.

13. Ein Computer (102), der Folgendes umfasst:
ein erstes Eingabegerät (110), das das mit Anmerkungen zu versehende medizinische Bild des Patienten abruft,
einen Prozessor (104), der das medizinische Eingangsbild mit einer Reihe von zuvor mit Anmerkungen versehenen medizinischen Bildern vergleicht, um Ähnlichkeiten zwischen dem medizinischen Eingangsbild und den einzelnen Bildern der Reihe von zuvor mit Anmerkungen versehenen medizinischen Bildern zu ermitteln, und Ermitteln des Bilds mit der jeweiligen vorab festgelegten Anmerkung sowie mit der größten Ähnlichkeit mit dem medizinischen Eingangsbild für die einzelnen vorab festgelegten Anmerkungen beruhend auf den ermittelten Ähnlichkeiten aus der Reihe der zuvor mit Anmerkungen versehenen medizinischen Bilder;
eine Anzeige (112) für das visuelle Darstellen des ermittelten Bilds für die einzelnen vorab festgelegten Anmerkungen gemeinsam mit den einzelnen vorab festgelegten Anmerkungen; und
ein zweites Eingabegerät zum Abrufen eines Eingangssignals, das eines der angezeigten Bilder und die angezeigten vorab festgelegten Anmerkungen angibt, wobei der Prozessor das medizinische Eingangsbild mit dem anhand des Eingangssignals ermittelten angezeigten Bild als Anmerkung versieht sowie einen elektronischen Bericht für das medizinische Bild generiert, der die angezeigte, anhand des Eingangssignals ermittelte Anmerkung enthält.

14. Der Computer gemäß Anspruch 13, wobei der Prozessor für jede der vorab festgelegten Anmerkungen eine Reihe mit mindestens einem zuvor mit Anmerkungen versehenen medizinischen Bild mit der größten Ähnlichkeit mit dem medizinischen Eingangsbild ermittelt.

15. Ein von einem Computer lesbares Speichermedium (108), das mit von einem Computer lesbaren Anweisungen codiert ist, die beim Ausführen den Prozessor (104) dazu veranlassen, die Schritte die Methode gemäß Anspruch 1 auszuführen.

## Revendications

1. Procédé mis en œuvre par ordinateur de création d'un rapport d'image médicale formaté électroniquement comportant une annotation d'image, comprenant :
la réception d'une image d'entrée médicale, d'un patient, à annoter ;
la comparaison de l'image d'entrée médicale avec un ensemble d'images médicales précédemment annotées pour déterminer des similitudes entre l'image d'entrée médicale et chaque image dans l'ensemble d'images médicales précédemment annotées ;
l'identification, pour chaque annotation d'une pluralité d'annotations prédéterminées, en fonction des similitudes déterminées, à partir de l'ensemble d'images médicales précédemment annotées l'image comportant ladite chaque annotation prédéterminée et présentant la plus grande similitude avec l'image d'entrée médicale ;
l'affichage visuel, pour chaque annotation de la pluralité d'annotations prédéterminées, de l'image identifiée pour ladite chaque annotation prédéterminée comportant ladite chaque annotation prédéterminée ;
la réception d'un signal identifiant l'une des images affichées et les annotations prédéterminées affichées ;
l'annotation de l'image d'entrée médicale avec l'image affichée identifiée par le signal d'entrée ; et
la génération, dans un format électronique, d'un rapport pour l'image d'entrée médicale, lequel comprend l'annotation affichée identifiée par le signal d'entrée.

2. Procédé selon la revendication 1, dans lequel l'identification identifie, pour chaque annotation de la pluralité d'annotations prédéterminées, un ensemble d'au moins une image médicale précédemment annotée présentant la plus grande similitude avec l'image d'entrée médicale.

3. Procédé selon la revendication 2, dans lequel les au moins deux images précédemment annotées présentent respectivement une similitude supérieure à un niveau de similitude seuil prédéterminé.

4. Procédé selon la revendication 2, dans lequel les au moins deux images médicales précédemment annotées présentent une similitude dans une plage de pourcentage de similitudes prédéterminée.

5. Procédé selon l'une quelconque des revendications 1 à 4, dans lequel l'ensemble d'images médicales précédemment annotées comprend des images médicales annotées précédemment pour d'autres patients.

6. Procédé selon l'une quelconque des revendications 1 à 5, dans lequel l'ensemble d'images médicales précédemment annotées comprend au moins une image médicale annotée correspondant à chaque annotation de la pluralité d'annotations prédéterminées.

7. Procédé selon l'une quelconque des revendications 1 à 6, dans lequel une totalité de l'image d'entrée médicale et une totalité de chaque image de l'ensemble d'images médicales précédemment annotées sont comparées.

8. Procédé selon la revendication 7, dans lequel les images sont comparées par pixel ou par voxel ou par sous-groupe par pixel ou par sous-groupe par voxel.

9. Procédé selon l'une quelconque des revendications 1 à 6, comprenant en outre :
la réception d'un signal indiquant une sous-zone de l'image d'entrée médicale, dans lequel seule la sous-zone de l'image d'entrée médicale et une sous-zone correspondante de chaque image de l'ensemble d'images médicales précédemment annotées sont comparées.

10. Procédé selon la revendication 9, comprenant en outre :
la segmentation de la sous-zone de l'image d'entrée médicale et de la sous-zone correspondante de chaque image de l'ensemble d'images médicales précédemment annotées ; et
la comparaison de la sous-zone segmentée de l'image d'entrée médicale et de la sous-zone segmentée de chaque image de l'ensemble d'images médicales précédemment annotées.

11. Procédé selon l'une quelconque des revendications 7 à 10, comprenant en outre :
la génération d'un vecteur de caractéristiques quantitatives pour chaque annotation de l'image d'entrée médicale et pour chaque image de l'ensemble d'images médicales précédemment annotées, dans lequel la comparaison comprend la comparaison des vecteurs de caractéristiques quantitatives.

12. Procédé selon la revendication 11, dans lequel le vecteur de caractéristiques quantitatives comprend des caractéristiques numériques descriptives d'une taille et/ou d'une position et/ou d'une luminosité et/ou d'un contraste et/ou d'une forme et/ou d'une texture.

13. Appareil informatique (102), comprenant :
un premier dispositif d'entrée (110), lequel reçoit une image d'entrée médicale, d'un patient, à annoter ;
un processeur (104), lequel compare l'image d'entrée médicale avec un ensemble d'images médicales précédemment annotées pour déterminer les similitudes entre l'image d'entrée médicale et chaque image dans l'ensemble d'images médicales précédemment annotées, et identifie, pour chaque annotation d'une pluralité d'annotations prédéterminées, en fonction des similitudes déterminées, à partir de l'ensemble d'images médicales précédemment annotées, l'image comportant ladite chaque annotation prédéterminée et présentant la plus grande similitude avec l'image d'entrée médicale ;
un affichage (112), lequel affiche visuellement, pour chaque annotation de la pluralité d'annotations prédéterminées, l'image identifiée pour ladite chaque annotation prédéterminée conjointement avec ladite chaque annotation prédéterminée ; et
un second dispositif d'entrée, lequel reçoit un signal d'entrée identifiant l'une des images affichées et les annotations prédéterminées affichées, dans lequel le processeur annote l'image d'entrée médicale avec l'image affichée identifiée par le signal d'entrée et génère, dans un format électronique, un rapport pour l'image d'entrée médicale, lequel comprend l'annotation affichée identifiée par le signal d'entrée.

14. Appareil informatique selon la revendication 13, dans lequel le processeur identifie, pour chaque annotation de la pluralité d'annotations prédéterminées, un ensemble d'au moins une image médicale précédemment annotée présentant la plus grande similitude avec l'image d'entrée médicale.

15. Support d'enregistrement lisible par ordinateur (108) codé à l'aide des instructions lisibles par ordinateur, lequel, lorsqu'il est exécuté par un processeur (104), amène le processeur à mettre en œuvre le procédé selon la revendication 1.
